# EUROPÄISCHE PATENTSCHRIFT

(11) **EP 0 701 691 B1**
(45) Veröffentlichungstag und Bekanntmachung des Hinweises auf die Patenterteilung: **05.03.1997**
(21) Anmeldenummer: 94915508.9
(22) Anmeldetag: 21.05.1994
(51) Int. Cl.: G01N 27/30, G01N 27/28, G01N 27/49

(54) **ELEKTROCHEMISCHER SENSOR**
ELECTROCHEMICAL SENSOR
DETECTEUR ELECTROCHIMIQUE

(30) Priorität: 03.06.1993 DE 4318519
(43) Veröffentlichungstag der Anmeldung: 20.03.1996
(73) Patentinhaber: FRAUNHOFER-GESELLSCHAFT ZUR FÖRDERUNG DER ANGEWANDTEN FORSCHUNG E.V., 80636 München (DE)
(72) Erfinder: HINTSCHE, Rainer, D-10199 Berlin (DE); PAESCHKE, Manfred, D-16352 Basdorf (DE); SCHNAKENBERG, Uwe, D-10559 Berlin (DE); WOLLENBERGER, Ulla, D-13187 Berlin (DE)
(86) Internationale Anmeldenummer: DE9400598
(87) Internationale Veröffentlichungsnummer: WO9429708

(56) Entgegenhaltungen:
- WO-A-90/15323
- WO-A-93/06237
- SENSORS AND ACTUATORS, Bd.15, Nr.4, Dezember 1988, LAUSANNE CH Seiten 337 - 345 SINCLAIR YEE, ET AL. 'MINIATURE LIQUID JUNCTION REFERENCE ELECTRODE WITH MICROMACHINED SILICON CAVITY'
- ANALYTICAL CHEMISTRY, Bd.62, 1990, COLUMBUS US Seiten 407 - 409 TOMOKAZU MATSUE, ET AL. 'MULTICHANNEL ELECTROCHEMICAL DETECTION SYSTEM FOR FLOW ANALYSIS'
- ANALYTICAL CHEMISTRY, Bd.64, 1992, COLUMBUS US Seiten 1118 - 1127 I. FRITSCH-FAULES, ET AL. 'USE OF MICROELECTRODE ARRAYS TO DETERMINE CONCENTRATION PROFILES OF REDOX CENTERS IN POLYMER FILMS' in der Anmeldung erwähnt
- ANALYTICAL CHEMISTRY, Bd.62, 1990, COLUMBUS US Seiten 447 - 452 OSAMU NIWA, ET AL. 'ELECTROCHEMICAL BEHAVIOR OF REVERSIBLE REDOX SPECIES AT INTERDIGITATED ARRAY ELECTRODES WITH DIFFERENT GEOMETRIES: CONSIDERATION OF REDOX CYCLING AND COLLECTION EFFICIENCY' in der Anmeldung erwähnt
- ANALYTICAL CHEMISTRY, Bd.64, 1992, COLUMBUS US Seiten 1118 - 1127 I. FRITSCH-FAULES, ET AL. 'USE OF MICROELECTRODE ARRAYS TO DETERMINE CONCENTRATION PROFILES OF REDOX CENTERS IN POLYMER FILMS'
- ANALYTICAL CHEMISTRY, Bd.62, 1990, COLUMBUS US Seiten 447 - 452 OSAMU NIWA, ET AL. 'ELECTROCHEMICAL BEHAVIOR OF REVERSIBLE REDOX SPECIES AT INTERDIGITATED ARRAY ELECTRODES WITH DIFFERENT GEOMETRIES: CONSIDERATION OF REDOX CYCLING AND COLLECTION EFFICIENCY'

## Beschreibung

### Technisches Gebiet

Die Erfindung betrifft einen elektrochemischen Sensor nach dem Oberbegriff des Anspruchs 1.

Elektrochemische Sensoren mit interdigitalen (d.h. fingerartig ineinandergreifenden) Mikroelektroden (siehe etwa WO-A-9 015 323 oder WO-A-9 306 237) sind für die chemische Analytik und Prozeßkontrolle auf verschiedenen Gebieten wie Biotechnologie, Umweltschutz, Gesundheitswesen einsetzbar. Sie weisen kleine Diffusionslängen für elektrochemisch aktive Moleküle auf, da die Abstände zwischen den einzelnen fingerartigen Elektrodenbereichen im µm- oder sub-µm-Bereich liegen.

### Stand der Technik

Bisher bekannt sind elektrochemische Sensoren mit miniaturisierten planaren Elektroden mit Geometrien oberhalb von 2 µm. Sie werden in Dünnfilmtechnologie hergestellt und als Transduktoren zur Detektion von chemischen oder biochemischen Substanzen verwendet.
Mit Elektrodengeometrien zwischen 20 und 100 µm werden nur Detektionseigenschaften der Elektroden erreicht, wie man sie auch bei konventionellen Elektroden aus dünnen Drähten findet (vgl. M. Suda et al., Proceedings Second World Congress on Biosensors, Genf, Schweiz 1992, p. 400; N.F. Sheppard, Jr. et al., Anal. Chem. 1993, 65, 1199 - 1202.
Aus L.D. Watson (Biosensors 3, 1987/88, 101-115) sind Anordnungen mit zwei parallelen und 5 µm breiten Metalldünnfilmbändern zur Leitfähigkeitsmessung bekannt.

Für die Beschreibung von Spannungsprofilen nutzten T. Matsue et al. (Anal. Chem. 62, 1990, 407-409) eine Anordnung von 16 Elektroden mit 1 mm Länge und 0,1 mm Breite.
Eine Anordnung von sphärischen Elektroden mit Mikrometerabmessungen in der Isolierung auf einer Metallfläche wird von B. Ross et al. (Sensors & Actuators B7, 1992, 758-762) beschrieben. Durch die elektrisch parallel miteinander verbundenen Mikroelektroden entsteht dabei nur ein Meßsignal bei voltammetrischen und chronoamperometrischen Messungen.
I. Fritsch-Faules et al. (Anal. Chem. 64, 1992, 1118-1127) verwenden eine Anordnung mit 4 µm breiten und 8 µm voneinander entfernten Elektroden für die Bestimmung von Konzentrationsprofilen von Redoxzentren in Polymerfilmen.
Ein elektrochemischer Sensor mit einem Paar interdigitaler Mikroelektroden ist aus O. Niwa et al. (Anal. Chem. 62, 1990, 447-452) bekannt. An den Mikroelektroden dieses Sensors mit Strukturbreiten zwischen 0,75 und 10 µm wurden erstmals Verstärkungseffekte durch elektrochemisches Rezyklisieren von reversiblen Redoxmolekülen aufgezeigt.
Alle beschriebenen Elektroden wurden jeweils in stationären Meßverfahren eingesetzt.

### Darstellung der Erfindung

Der Erfindung liegt die Aufgabe zugrunde, einen elektrochemischen Sensor anzugeben, der die elektrochemische Detektion von Molekülen mit höherer Nachweisempfindlichkeit ermöglicht und erweiterte Einsatzmöglichkeiten zur Erfassung chemischer Reaktionsabläufe bietet.

Die erfindungsgemäße Lösung dieser Aufgabe ist in Anspruch 1 angegeben. Weiterbildungen und besondere Ausgestaltungen der Erfindung sind Gegenstand der Unteransprüche.

Erfindungsgemäß weist der elektrochemische Sensor nach Anspruch 1 zwei oder mehr Paare interdigitaler Mikroelektroden mit Strukturbreiten unter 1 µm auf, die als Mikroelektrodenarray auf einem Substrat angeordnet sind. Die einzelnen Paare sind unabhängig voneinander mit elektrischen Potentialen beaufschlagbar, und die Meßeffekte an den einzelnen Elektroden sind unabhängig voneinander ableitbar. Unter Meßeffekten sind hier insbesondere amperometrische, potentiometrische oder impedimetrische Effekte zu verstehen. Vorzugsweise wird ein planares Substrat aus Glas, Saphir, Silizium oder Polymeren verwendet, wobei die Elektroden in Planartechnologie aufgebracht werden. Durch die Feinstrukturierung der Elektroden wird erreicht, daß die Abstände zwischen den interdigitalen, d.h. fingerartig angeordneten Mikroelektroden (z.B. Abstände von ca. 700 nm) klein werden gegenüber den Entfernungen, die nachzuweisende Moleküle während der Meßzeit zurücklegen. Dies ermöglicht es, das-gleiche Molekül mehrfach elektrochemisch zu erfassen, z.B. es wiederholt zu oxidieren und zu reduzieren, bevor es aus dem Elektrodenbereich wegdiffundiert ist.
Auf diese Weise erreicht man besonders vorteilhafte Verstärkungseffekte. Die erfindungsgemäße mehrfache Anordnung dieserart feinstrukturierter Elektrodenpaare führt in vorteilhafter Weise zur Multiplikation des eben beschriebenen Verstärkungseffektes, indem mit geeigneter Multielektrodenmeßtechnik ( z.B. mit einem rechnergestützten Multipotentiometer) sowohl simultane Messungen als auch die Wiedererfassung von Molekülspezies, die vorher an einem benachbarten interdigitalen Mikroelektrodenpaar erzeugt wurden, realisiert werden. Simultane Messung und Wiedererfassung führen zu einer signifikanten Verbesserung der analytischen Nachweisempfindlichkeit.
Zusätzliche vorteilhafte Einsatzmöglichkeiten des erfindungsgemäßen Sensors sind weiter unten beschrieben.

Die Mikroelektroden bestehen gemäß Anspruch 2 vorzugsweise aus dünnen Schichten von Edelmetallen und/oder potentialbildenden Metalloxiden (z.B. Silber-Silberhalogenid) oder Metallsalzen. Diese dünnen Schichten sind als Dünnfilmelektroden so in die Substratoberfläche eingelegt bzw. eingegraben, daß eine Planarisierung der Oberfläche und gleichzeitig eine mechanische Stabilisierung der Elektroden erreicht wird. Durch die Planarisierung wird die Diffusionscharakteristik zwischen zwei benachbarten Elektroden optimiert.

Eine besonders vorteilhafte Weiterbildung des erfindungsgemäßen Sensors besteht nach Anspruch 3 darin, daß sich die aktiven Flächen der Mikroelektroden in einem Mikrokanal mit Zu- und Abflußöffnung befinden, der auf das Substrat aufgebracht ist. Der Mikrokanal kann z.B. ein Kanal oder Graben aus geätztem oder laserstrukturiertem Silizium oder Polymeren sein, der auf das Substrat aufgeklebt oder gebondet ist. Das gesamte Mikroelektrodenarray ist somit in einem definierten und konstanten Raum geringen Volumens, dem erfindungsgemäßen Mikrokanal, angeordnet. Der besondere Vorteil dieser Anordnung ergibt sich aus dem konstanten und kleinen Volumen von wenigen Nanolitern, das die Mikroelektroden umgibt und hochempfindlich kleinste Veränderungen der zu untersuchenden Substanz zu messen gestattet. Bei in Kanalrichtung in Reihe angeordneten interdigitalen Mikroelektrodenpaaren (die fingerartigen Elektrodenbereiche sind senkrecht zur Kanalrichtung angeordnet) ergibt sich die Möglichkeit, gleichzeitig oder zeitversetzt an verschiedenen Stellen des Mikrokanals zu messen.

Als besondere Ausgestaltung ist nach Anspruch 4 der Mikrokanal als mechanisches Hilfsmittel zur Immobilisierung oder Rückhaltung von chemischen oder biochemischen Komponenten ausgebildet, indem z.B. durch schräge oder überstehende Kanalwände Polymerfilme mit immobilisierten Biokomponenten fixiert werden können. Durch siliziumgeätzte Gitter gelingt auch die Retention von chemisch beladenen Mikrokugeln, wobei der Mikrokanal in diesem Fall als Reaktorgefäß dient.

Nach Anspruch 5 sind auf dem Substrat zusätzlich zu den Mikroelektrodenarrays, z.B. als umgebende Fläche, weitere flächige Elektroden als Arbeits- oder Bezugselektroden angeordnet.

Eine Weiterbildung des erfindungsgemäßen elektrochemischen Sensors ist in Anspruch 6 angegeben. Die Weiterbildung betrifft die flüssigkeitsresistente Isolation der Leiterbahnen, die die Mikroelektroden mit Kontaktflächen am Rand des Substrats verbinden, durch eine isolierende Schicht, vorzugsweise aus Siliziumoxid, Siliziumnitrid oder Photolacken. Die isolierende Schicht ist so aufgebracht, daß die aktiven Flächen der Mikroelektroden für das analytische Medium und die Kontaktflächen für elektrische Kontaktierungen zugänglich bleiben.

Der erfindungsgemäße elektrochemische Sensor hat eine Vielzahl von Einsatzmöglichkeiten. So läßt er sich z.B. zur simultanen Vielfachmessung der gleichen Molekülspezies einsetzen. Wie in Anspruch 7 dargelegt, können durch Vielfachmessung des gleichen Meßeffektes mit der Mehrfachanordnung der interdigitalen Mikroelektroden die amperometrischen, potentiometrischen oder impedimetrischen Meßeffekte an den einzelnen Mikroelektroden ausgemittelt werden. Jeder Meßeffekt wird entsprechend der Zahl n der Mikroelektroden n-fach gemessen und somit gemäß der Quadratwurzel aus n das Signal-Rauschverhältnis bzw. die Empfindlichkeit der Gesamtmessung verbessert.

Eine weitere Verwendungsmöglichkeit des erfindungsgemäßen Sensors besteht gemäß Anspruch 8 in der gleichzeitigen oder zeitversetzten Detektion unterschiedlicher elektrochemischer Reaktionen oder zur Detektion des zeitlichen Verlaufs der gleichen oder unterschiedlicher elektrochemischer Reaktionen. Durch gleichzeitiges oder zeitversetztes Anlegen unterschiedlicher Potentiale an die einzelnen Mikroelektroden können verschiedene elektrochemische Prozesse, die an den jeweiligen Elektroden ablaufen, gleichzeitig oder zeitversetzt detektiert werden. Zeitversetzte Applikation der Potentiale an räumlich eng benachbarten Mikroelektroden bietet außerdem die vorteilhafte Möglichkeit, schnelle Reaktionen zeitlich verfolgen zu können. Das Meßverfahren der Pulspolarographie kann durch zeitlich alternierendes Anlegen der Potentiale an die Mikroelektroden ebenfalls angewendet werden.

Nach Anspruch 9 läßt sich der erfindungsgemäße Sensor zur Messung der Strömungsgeschwindigkeit einer analytischen Probe, der zeitlichen Probenveränderung oder von Durchmischungseffekten der Probe verwenden, indem elektrochemisch aktive Moleküle der Probe an verschiedenen Elektroden durch örtlich und/oder zeitlich versetzte Detektion gemessen werden. Ein wesentlicher Vorteil ergibt sich bei der Messung, wenn die Mikroelektroden in einem Mikrokanal gemäß Anspruch 3 mit definiertem und konstantem Volumen angeordnet sind. Vorteilhaft wird dabei an einer Elektrode eine Spezies erzeugt und an einer anderen stömungsabhängig gemessen. Durch das konstante Volumen können bei gestopptem Fluß auch die Probenveränderung mit der Zeit oder Durchmischungseffekte verfolgt werden.

Eine vorteilhafte Verwendung des erfindungsgemäßen Sensors besteht nach Anspruch 10 darin, die Aktivität von Enzymen, die bei Einsatz geeigneter Enzymsubstrate die Freisetzung eines elektrochemisch reversiblen Produktes katalysieren, mit hoher Empfindlichkeit nachzuweisen, indem eine Mikroelektrode eines interdigitalen Mikroelektrodenpaares so polarisiert wird, daß das reduzierte Reaktionsprodukt oxidiert wird, und die andere Mikroelektrode des gleichen interdigitalen Mikroelektrodenpaares so polarisiert wird, daß das oxidierte Reaktionsprodukt reduziert wird. Damit kann die Freisetzung reversibel oxidierbarer und reduzierbarer Produkte, insbesondere hydrolytischer Enzymreaktionen, sehr empfindlich durch die mehrfache Umsetzung des Produktes der Enzymreaktion detektiert werden. Dieser Einsatz des erfindungsgemäßen Sensors erlaubt die Bestimmung von Enzymsubstraten mit hoher Empfindlichkeit und ist für den hochempfindlichen Nachweis von Enzymen geeignet.
Das Enzym kann z.B. auch selbst Analyt sein oder als Marker in Immunotests oder DNA-Hybridisierungsmethoden dienen. Hierbei werden das oder die Enzyme in gelöster Form extern inkubiert oder dem Elektrodenraum (z.B. Mikrokanal) zugeführt oder sind bereits immobilisiert im Mikroreaktionsraum angeordnet.

Die erfindungsgemäße Anordnung wird im folgenden anhand der Ausführungsbeispiele und der Zeichnungen näher erläutert.

Dabei zeigen:
- Figur 1: schematisch ein Beispiel des elektrochemischen Sensors,
- Figur 2: schematisch einen Querschnitt (A-A') und einen Längsschnitt (B-B') durch die Elektrodenanordnung des elektochemischen Sensors aus Fig. 1 mit aufgebrachtem Mikrokanal (in Fig. 1 nicht gezeigt),
- Figur 3: Meßkurven der Oxidation von Ferrocyanid, und
- Figur 4: Meßkurven des Nachweises von p-Aminophenol.

### Ausführungsbeispiel

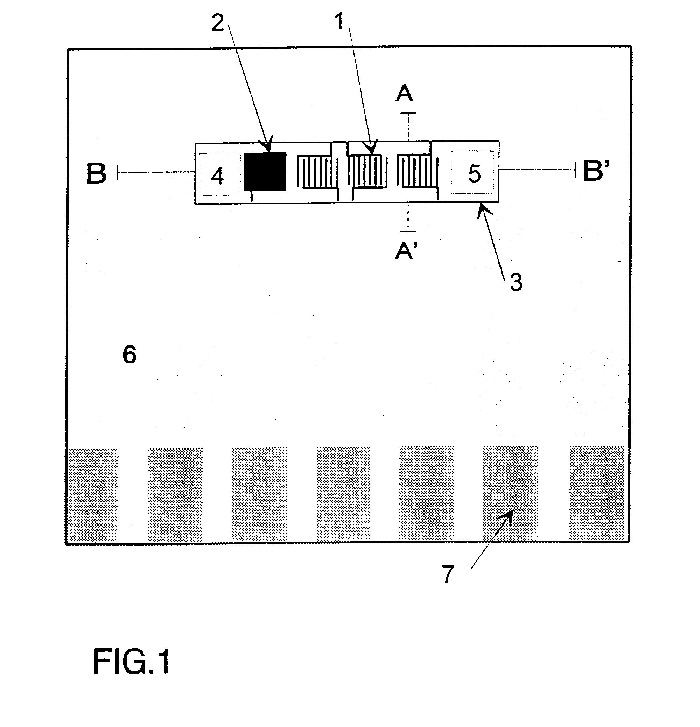

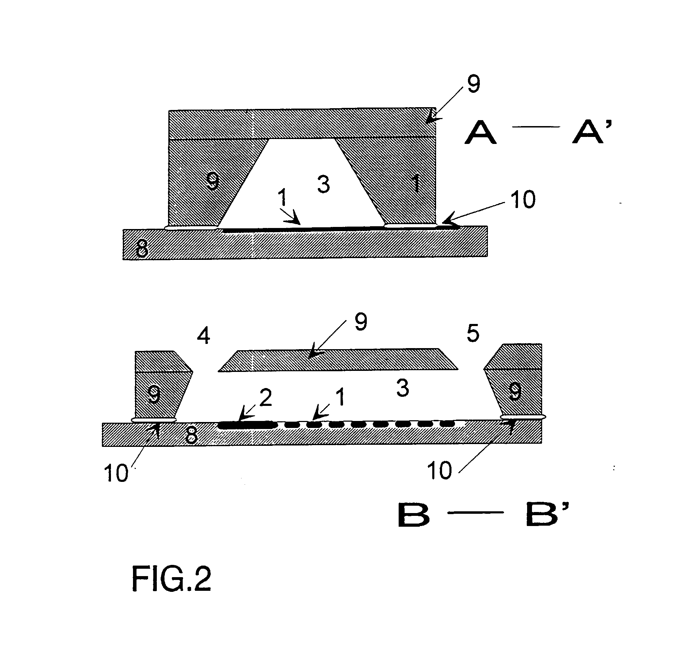

Ein Ausführungsbeispiel für den erfindungsgemäßen elektrochemischen Sensor ist in Figur gezeigt. Die interdigitalen Mikroelektroden 1 sind hier paarweise in einer Reihe gemeinsam mit der Referenzelektrode 2 auf einem planaren Silizium-Chip 8 angeordnet. Die Leiterbahnen von den Mikroelektroden 1 und der Referenzelektrode 2 zu den elektrischen Kontaktflächen 7 sind durch eine isolierende Schicht 6 abgedeckt.
Ein Querschnitt entlang der Achse A-A' und ein Längsschnitt entlang der Achse B-B' aus Figur 1 ist in Figur 2 mit aufgesetztem Mikrokanal 3 gezeigt. Der Mikrokanal 3 besteht in diesem Beispiel aus einem Silizium-Chip 9 mit anisotrop geätztem Graben, der auf den die Mikroelektroden 1 enthaltenden Silizium-Chip 8 aufgeklebt ist. Zwischen den beiden Silizium-Chips 8 und 9 befindet sich die Kleb- bzw. Dichtungsschicht 10. Zu- und Abflußöffnung 4 und 5 des Mikrokanals 3 sind im Längsschnitt zu erkennen.

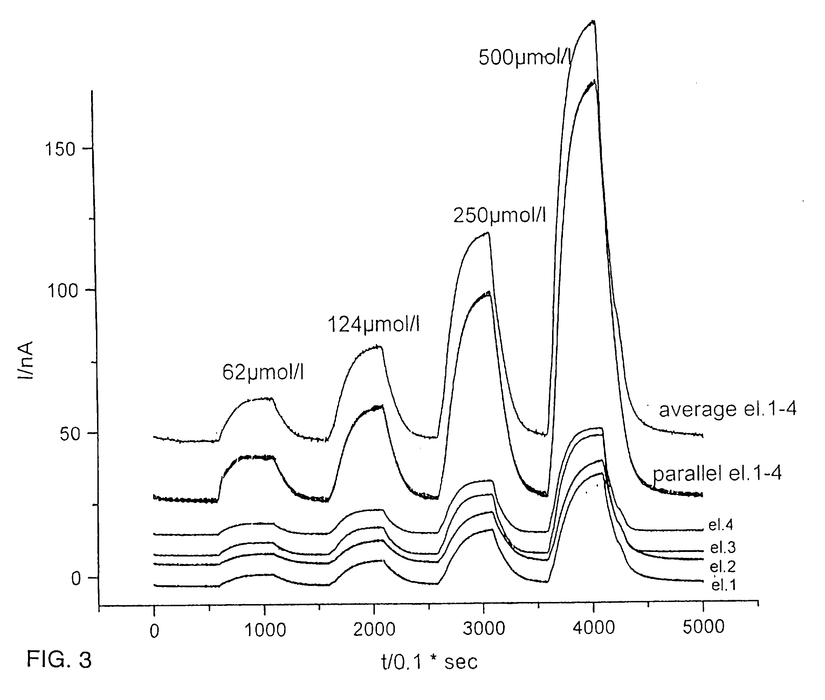

Figur 3 zeigt mit dem erfindungsgemäßen Sensor aufgenommene Meßkurven der Oxidation von Ferrocyanid. Die einzelnen Maxima ergeben sich nach Zugabe von 62, 124, 250 und 500 µmol/l Ferrocyanid. Dargestellt sind die simultan erfaßten Meßkurven (I(t)) jeder einzelnen von vier Mikroelektroden (el.1,el.2,el.3,el.4), die Meßkurve der Parallelschaltung der vier Elektroden (parallel el.1-4) und die ausgemittelte Meßkurve aus der Simultanmessung mit den vier Elektroden (average el.1-4). Das geringere Signal-Rauschverhältnis bei der mit dem erfindungsgemäßen Sensor möglichen simultanen Vielfachmessung ist deutlich zu erkennen.

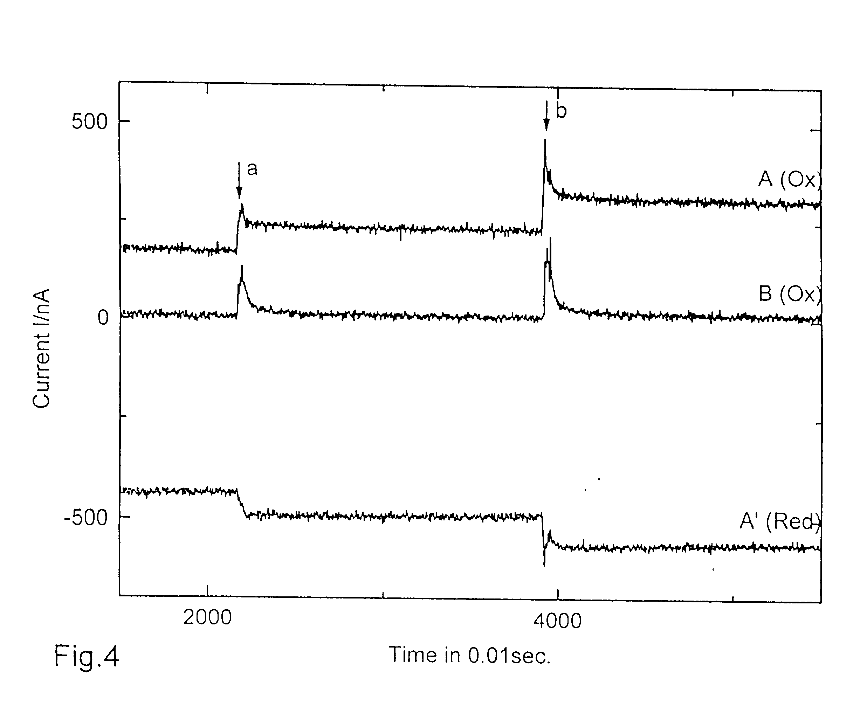

Der Verstärkungseffekt der interdigitalen Mikroelektrodenanordnung ist in Figur 4 am Beispiel von Meßkurven (1(t)) des Nachweises von p-Aminophenol mit wiederholter Oxidation/Reduktion an einem interdigitalen Mikroelektrodenpaar (A(Ox) und A'(Red)) dargestellt. Zum Vergleich dazu ist eine Meßkurve (B(Ox)) gezeigt, die bei konventioneller Oxidation (zweite Elektrode ohne Funktion) am gleichen Elektrodenpaar gemessen wurde. (a,b: jeweils Zugabe von 50 µmol/l p-Aminophenol). Die deutlichen Unterschiede in der Signalhöhe zeigen den Vorteil der interdigitalen Mikroelektrodenanordnung.

Im folgenden werden zwei Ausführungsbeispiele zur Verwendung des erfindungsgemäßen elektrochemischen Sensors angegeben.

Das erste Ausführungsbeispiel betrifft die empfindliche Bestimmung der Aktivität von alkalischer Phosphatase. Der in den Figuren 1 und 2 gezeigte elektrochemische Sensor wird in ein Fließsystem eingesetzt. Mit einem Multipotentiostaten wird eine Elektrode eines interdigitalen Elektrodenpaares gegenüber einer Silber/Silberchlorid (gesättigte KCI) - Referenzelektrode (im Abfluß positioniert) auf 250 mV polarisiert. An die jeweils andere Elektrode des Elektrodenpaares wird ein Potential von -50 mV angelegt. In gleicher Weise werden an alle anderen Elektrodenpaare des interdigitalen Mikroelektrodenarrays unabhängig voneinander die entsprechenden anodischen oder kathodischen Potentiale gelegt. Die Meßlösung, bestehend aus 0,1 mol/l Phosphatpufferlösung (pH 7,0) mit 0,1 mol/l KCI, wird mit 0,8 ml/min Fließgeschwindigkeit durch die Elektrodenmikrokammer (Mikrokanal) geführt. Die Grundströme jeder Elektrode werden simultan aber unabhängig voneinander abgeleitet. Nach Injektion von 1 µmol/l p-Aminophenol werden an jeder einzelnen Anode die Erhöhung der Oxidationsströme und an jeder einzelnen Kathode die Veränderung der Reduktionsströme registriert (zum Vergleich siehe Figur 1). Die Meßdaten werden rechnerisch zu rauschverminderten gemittelten Meßsignalen verarbeitet. Durch Registrierung und Verarbeitung der Stromänderung nach Injektion von 2, 5 und 10 µmol/l p-Aminophenol wird eine Eichkurve erstellt. Gleichzeitig werden in externen temperierten Meßgefäßen Proben mit alkalischer Phosphatase mit 5 mmol/l p-Aminophenylphosphat in 1 mol/l Diethanolamin-Puffer (pH 9,8; mit 0,5 mmol/l MgCl₂, 37°C) inkubiert. Nach 1 Minute Inkubationszeit werden 100 µl der Reaktionslösung in den fließenden Puffer der Meßanordnung injiziert. Die Veränderung der amperometrischen Elektrodensignale wird wie oben beschrieben einzeln abgeleitet und verarbeitet. Die Enzymaktivität der Meßprobe, definiert als Bildungsgeschwindigkeit von p-Aminophenol aus p-Aminophenylphosphat (1 U = 1 µmol/min), wird mit Hilfe der zuvor erstellten Eichkurve berechnet.

Im zweiten Ausführungsbeispiel wird die Aktivität von alkalischer Phosphatase, die auf einem mikrodispersen Träger immobilisiert ist, mit der im vorhergehenden Ausführungsbeispiel beschriebenen Meßanordnung bestimmt. Die Träger werden dazu in der beschriebenen Mikrokammer durch ein geätztes Siliziumgitter zurückgehalten. Nach der p-Aminophenylphosphat-Zugabe erfolgt die Registrierung der Bildung von p-Aminophenol. Die Messung, Eichung und Auswertung erfolgt analog zum vorhergehenden Ausführungsbeispiel.

## Patentansprüche

1. Elektrochemischer Sensor mit zwei oder mehr Paaren interdigitaler Mikroelektroden (1), die Strukturbreiten im sub-µm-Bereich aufweisen und an der Oberfläche eines Substrates (8) angeordnet sind, wobei die einzelnen Paare unabhängig voneinander mit elektrischen Potentialen beaufschlagbar, und Meßeffekte an den einzelnen Elektroden unabhängig voneinander ableitbar sind,
**dadurch gekennzeichnet,**
daß durch einen gemeinsamen Elektrolyten eine elektrische Verbindung zwischen allen Elektroden herstellbar ist, und daß zumindest zwei Paare der interdigitalen Mikroelektroden mit fingerförmigen Ausläufern als Mikroelektrodenarray so in einer Richtung hintereinander angeordnet sind, daß ihre fingerförmigen Ausläufer senkrecht zu dieser Richtung verlaufen.

2. Elektrochemischer Sensor nach Anspruch 1,
**dadurch gekennzeichnet,**
daß die Mikroelektroden aus dünnen Schichten von Edelmetallen und/oder potentialbildenden Metalloxiden oder Metallsalzen bestehen, die so in die Substratoberfläche eingegraben sind, daß eine Planarisierung der Oberfläche errreicht wird.

3. Elektrochemischer Sensor nach Anspruch 1 oder 2,
**dadurch gekennzeichnet,**
daß sich die aktiven Flächen der Mikroelektroden in einem Mikrokanal 3 mit Zu- und Abflußöffnung (4, 5) befinden, der auf das Substrat aufgebracht ist.

4. Elektrochemischer Sensor nach einem der Ansprüche 1 bis 3,
**dadurch gekennzeichnet,**
daß der Mikrokanal so ausgebildet ist, daß er als mechanisches Hilfsmittel für die Immobilisierung von chemisch oder biologisch aktiven Substanzen genutzt werden kann.

5. Elektrochemischer Sensor nach einem der Ansprüche 1 bis 4,
**dadurch gekennzeichnet,**
daß auf dem Substrat zusätzlich weitere flächige Elektroden (2) als Arbeits-oder Bezugselektroden angeordnet sind.

6. Elektrochemischer Sensor nach einem der Ansprüche 1 bis 5,
**dadurch gekennzeichnet,**
daß Leiterbahnen, die von den Mikroelektroden zu Kontaktflächen führen, durch eine isolierende Schicht, vorzugsweise aus Siliziumoxid oder Siliziumnitrid oder Photolacken, so bedeckt sind, daß nur die aktiven Mikroelektrodenflächen für analytische Medien und die Kontaktflächen für elektrische Kontaktierungen offen zugänglich sind.

7. Verwendung des elektrochemischen Sensors nach einem der Ansprüche 1 bis 6 zur Vielfachmessung des gleichen Meßeffektes, wobei die Mehrfachanordnung der interdigitalen Mikroelektroden zur Ausmittelung der amperometrischen, potentiometrischen oder impedimetrischen Meßeffekte an den einzelnen Mikroelektroden genutzt wird.

8. Verwendung des elektrochemischen Sensors nach einem der Ansprüche 1 bis 6 zur Detektion unterschiedlicher elektrochemischer Reaktionen oder zur Detektion des zeitlichen Verlaufs elektrochemischer Reaktionen, indem an die einzelnen Mikroelektroden gleichzeitig oder zeitversetzt und/oder zeitlich alternierend unterschiedliche Potentiale angelegt werden.

9. Verwendung des elektrochemischen Sensors nach einem der Ansprüche 1 bis 6 zur Messung der Strömungsgeschwindigkeit einer analytischen Probe, der zeitlichen Probenveränderung oder von Durchmischungseffekten, indem elektrochemisch aktive Moleküle der Probe an verschiedenen Elektroden durch örtlich und/oder zeitlich versetzte Detektion gemessen werden.

10. Verwendung des elektrochemischen Sensors nach einem der Ansprüche 1 bis 6 zum Nachweis der Aktivität von Enzymen mit hoher Empfindlichkeit, die bei Einsatz geeigneter Enzymsubstrate die Freisetzung eines elektrochemisch reversiblen Produktes katalysieren, indem eine Mikroelektrode eines interdigitalen Mikroelektrodenpaares so polarisiert wird, daß das reduzierte Reaktionsprodukt oxidiert wird, und die andere Mikroelektrode des gleichen interdigitalen Mikroelektrodenpaares so polarisiert wird, daß das oxidierte Reaktionsprodukt reduziert wird.

## Claims

1. Electrochemical sensor with two or more pairs of interdigital microelectrodes (1) which have structural widths in the sub-µ range and are arranged on the surface of a substrate (8), the individual pairs being chargeable independently of one another with electrical potentials, and measurement effects may be derived independently from one another at the individual electrodes,
**characterised in that**
an electrical connection may be established between all the electrodes by means of a common electrolyte, and in that at least two pairs of the interdigital microelectrodes are disposed with finger-shaped extensions as a microelectrode array in a direction one behind the other in such a way that their finger-shaped extensions extend vertically to this direction.

2. Electrochemical sensor according to claim 1,
**characterised in that**
the microelectrodes comprise thin layers of noble metals and/or potential-forming metallic oxides or metal salts which are engraved in the substrate surface in such a way that planarisation of the surface is achieved.

3. Electrochemical sensor according to claim 1 or 2,
**characterised in that**
the active surfaces of the microelectrodes are located in a microchannel 3 with inflow- and outflow opening (4, 5), which is applied to the substrate.

4. Electrochemical sensor according to one of claims 1 to 3,
**characterised in that**
the microchannel is so formed that it can be used as a mechanical auxiliary means for immobilising chemically or biologically active substances.

5. Electrochemical sensor according to one of claims 1 to 4,
**characterised in that**
further surface electrodes (2) are disposed on the substrate as operational or reference electrodes.

6. Electrochemical sensor according to one of claims 1 to 5,
**characterised in that**
strip conductors, which lead from the microelectrodes to contact surfaces, are covered by an insulating layer, preferably of silicon oxide or silicon nitride or photosensitive resists, in such a way that only the active microelectrode surfaces are openly accessible for analytic media, and the contact surfaces for electrical contacts.

7. Utilisation of an electrochemical sensor according to one of claims 1 to 6 for multiple measurement of the same measurement effect, the multiple arrangement of the interdigital electrodes being used to derive the amperometric, potentiometric or impedimetric measurement effects at the individual microelectrodes.

8. Utilisation of an electrochemical sensor according to one of claims 1 to 6 for detecting different electrochemical reactions or for detecting the time history of electrochemical reactions, different potentials being applied to the individual microelectrodes simultaneously or offset in time and/or alternating chronologically.

9. Utilisation of an electrochemical sensor according to one of claims 1 to 6 for measuring the current speed of an analytical sample, the alteration in time of samples or mixing effects, electrochemically active molecules of the sample being measured at different electrodes by locally and/or chronologically offset detection.

10. Utilisation of an electrochemical sensor according to one of claims 1 to 6 for indicating with high sensitivity the activity of enzymes which, when appropriate enzyme substrates are used, catalyse the liberation of an electrochemically reversible product, one microelectrode of an interdigital microelectrode pair being so polarised that the reduced reaction product is oxidised, and the other microelectrode of the same interdigital microelectrode pair being so polarised that the oxidised reaction product is reduced.

## Revendications

1. Détecteur électrochimique avec deux ou plusieurs paires de microélectrodes (1) interdigitales qui comportent des largeurs structurelles au niveau du sous-µm et sont disposées à la surface d'un substrat (8), tandis que les paires individuelles peuvent être chargées indépendamment l'une de l'autre avec des potentiels électriques, et que des effets de mesure sont déductibles sur les électrodes individuelles indépendamment les uns des autres,
caractérisé en ce que
grâce à un électrolyte commun on peut produire une connexion électrique entre toutes les électrodes et en ce qu'au moins deux paires des microélectrodes interdigitales sont disposées avec des ramifications en forme de doigt comme système de microélectrodes dans une direction telle que leurs ramifications en forme de doigt se développent perpendiculairement à cette direction.

2. Détecteur électrochimique selon la revendication 1,
caractérisé en ce que
les microélectrodes sont composées de couches minces de métaux nobles et/ou d'oxydes métalliques ou sels métalliques formant un potentiel qui sont gravées dans la surface du substrat, de manière à obtenir une planéité de la surface.

3. Détecteur électrochimique selon les revendications 1 ou 2,
caractérisé en ce que
les surfaces actives des microélectrodes se trouvent dans un microcanal (3) avec une ouverture d'amenée et de départ (4, 5) qui est appliquée au substrat.

4. Détecteur électrochimique selon une des revendications 1 à 3,
caractérisé en ce que
le microcanal est constitué de manière, qu'il puisse être utilisé comme agent auxiliaire mécanique pour l'immobilisation de substances chimiquement ou biologiquement actives.

5. Détecteur électrochimique selon une des revendications 1 à 4,
caractérisé en ce que
sur le substrat sont disposées en plus d'autres électrodes plates comme électrodes de travail ou de référence (2).

6. Détecteur électrochimique selon une des revendications 1 à 5,
caractérisé en ce que
des pistes conductrices, qui vont des microélectrodes aux surfaces de contact, sont recouvertes par une couche isolante, de préférence en oxyde de silicium ou nitrure de silicium ou photolaques, de sorte que seules les surfaces actives des microélectrodes sont ouvertement accessibles aux milieux analytiques et les surfaces de contact aux liaisons électriques.

7. Utilisation du détecteur électrochimique, selon une des revendications 1 à 6 pour la mesure multiple du même effet de mesure, dans lequel la disposition répétée des microélectrodes interdigitales est utilisées pour l'homogénéisation des effets de mesure ampérométriques, potentiométriques ou impédimétriques sur les microélectrodes individuelles.

8. Utilisation du détecteur électrochimique selon une des revendications 1 à 6, pour la détection de réactions électrochimiques différentes ou pour la détection du développement dans le temps de réactions électrochimiques, en appliquant sur les microélectrodes individuelles en même temps ou avec décalage dans le temps et/ou en alternant dans le temps des potentiels différents.

9. Utilisation du détecteur électrochimique selon une des revendications 1 à 6 pour la mesure de la vitesse d'écoulement d'un échantillon analytique, de la modification de l'échantillon dans le temps ou des effets de mélange intime, en soumettant à des mesures des molécules électrochimiquement actives de l'échantillon sur différentes électrodes au moyen d'une détection localisée et/ou décalée dans le temps.

10. Utilisation du détecteur électrochimique selon une des revendications 1 à 6 pour le décèlement de l'activité d'enzymes à sensibilité élevée, qui catalysent lors de l'utilisation de substrats d'enzymes appropriés la libération d'un produit électrochimiquement réversible, en polarisant une microélectrode d'une paire de microélectrodes interdigitales de sorte, que le produit réactionnel soit oxydé, et en polarisant l'autre microélectrode de la même paire de microélectrodes interdigitales de sorte que le produit réactionnel oxydé soit réduit.
